# DEMANDE DE BREVET EUROPEEN

(11) **EP 2 163 218 A1**
(43) Date de publication de la demande: **17.03.2010**
(21) Numéro de dépôt: 08370019.5
(22) Date de dépôt: 16.09.2008
(51) Int. Cl.: A61B 18/20, A61N 5/06

(54) **Appareil de traitement d'une partie de corps humain ou animal, comportant un instrument permettant de délivrer et/ou un instrument permettant d'aspirer localement des doses de traitement et des moyens de controle de dosimétrie**

(71) Demandeur: Osyris Medical, 59260 Hellemmes Lille (FR)
(72) Inventeur: Zemmouri, Jaouad, 59242 Genech (FR); Rochon, Philippe, 59182 Loffre (FR); Lefevre, Liliane, 59000 Lille (FR); Mordon, Serge, 59420 Mouvaux (FR); Wassmer, Benjamin, 59139 Wattignies (FR)
(74) Mandataire: Matkowska, Franck

(57) **Abrégé**

L'appareil de traitement d'une partie de corps humain ou animal, comporte un instrument (1) qui permet de délivrer des doses de traitement via une sortie (12a) de l'instrument et/ou comporte un instrument (1) qui permet d'aspirer des doses de traitement via une entrée de l'instrument. Il comprend en outre :
- des moyens (2) de localisation, dans un repère tridimensionnel prédéfini (X,Y,Z), de la position absolue instantanée 3D de la sortie (12a) de l'instrument délivrant les doses et/ou de l'entrée de l'instrument (12) aspirant les doses, lesquels moyens (2) de localisation sont aptes à délivrer des données P'[(x'(t), y'(t), z'(t)] codant la position absolue instantanée 3D de la sortie (12a) et/ou de l'entrée de l'instrument (12), et
- des moyens électroniques (3) de contrôle de dosimétrie qui sont aptes à calculer au moins un paramètre de dosimétrie à partir au moins des données de localisation P'[(x'(t), y'(t), z'(t)] qui sont délivrées par les moyens (2) de localisation.

## Description

### Domaine technique

La présente invention concerne le domaine du traitement du corps humain ou animal au moyen d'un instrument qui permet de délivrer localement des doses de traitement à une partie de corps humain ou animal, ou qui au contraire permet d'aspirer localement des doses de traitement dans une partie de corps humain ou animal, tel que par exemple un traitement de liposuccion. Dans le domaine du traitement du corps humain ou animal au moyen d'un instrument qui permet de délivrer localement des doses de traitement, l'invention concerne plus particulièrement, mais non exclusivement, le domaine du traitement d'une partie de corps humain ou animal par irradiation cutanée, sous-cutanée ou intra-cutanée au moyen d'un rayonnement électromagnétique, les doses de traitement correspondant dans ce cas aux énergies dudit rayonnement électromagnétique délivrées à différentes positions de l'instrument dans la zone ou le volume traité. Dans le cadre de l'invention, et selon le type d'instrument utilisé, le traitement peut être un traitement thérapeutique, prophylactique ou cosmétique de type non invasif, ou être un traitement thérapeutique, prophylactique ou cosmétique invasif, par exemple sous-cutané ou intra-cutané, tels par exemple la lipolyse, les traitements endoveineux, le remodelage, la cicatrisation de la peau par chauffage du collagène présent dans le derme, la liposuccion.

### Art antérieur

Il existe de nombreux traitements différents au cours desquels on délivre localement une dose de traitement à une partie de corps humain ou animal, à des fins cosmétiques, thérapeutiques ou prophylactiques de ladite partie de corps humain ou animal, et en utilisant un instrument médical spécifique, encore communément désigné « pièce à main », et adapté notamment à la nature des doses de traitement à délivrer.

La dose de traitement peut être par exemple une dose de rayonnement électromagnétique ; dans ce cas, la dose délivrée correspond à l'énergie du rayonnement électromagnétique appliqué à une position donnée de l'instrument dans une zone ou volume de traitement. La dose de traitement peut être par exemple une dose d'une substance ou produit chimique administré localement à ladite partie de corps humain ou animal ; dans ce cas, la dose délivrée correspond à la quantité de substance ou produit chimique administrée à chaque position de l'instrument.

On peut classer ces différents traitements thérapeutiques, prophylactiques, ou cosmétiques en deux catégories différentes, selon qu'ils sont de type invasif ou de type non invasif.

Parmi les traitements de type non invasif, on peut citer tous les traitements thérapeutiques, prophylactiques, ou cosmétiques basés sur une irradiation intra-cutanée ou sous-cutanée de la zone à traiter au moyen d'un rayonnement électromagnétique, et notamment sur une irradiation au moyen d'un rayonnement électromagnétique produit par exemple dans le domaine de longueurs d'onde du visible en utilisant un faisceau laser continu ou pulsé à différentes puissances. Dans ces traitements intra-cutanés ou sous-cutanés, on introduit le rayonnement électromagnétique dans le derme ou sous le derme, jusqu'à la zone où volume à traiter, au moyen par exemple d'une aiguille creuse ou d'une canule, dans laquelle est introduite une fibre optique reliée à une source de rayonnement électromagnétique adaptée, par exemple à un laser. Puis on effectue le traitement en tirant, de manière continue ou de manière discontinue, sur l'ensemble canule/fibre optique ou aiguille/fibre optique, et en actionnant la source laser, de manière à effectuer des tirs lasers à différentes positions de l'extrémité distale d'émission de la fibre optique lors du mouvement continu ou discontinu de retrait de l'ensemble canule/fibre optique ou aiguille/fibre optique.

Plus particulièrement, parmi les traitements par irradiation électromagnétique sous-cutanée, on peut citer principalement, mais de manière non exhaustive, la lipolyse qui consiste à détruire, notamment par effets thermiques, les cellules adipeuses présentes dans l'hypoderme en introduisant dans l'hypoderme, à différentes profondeurs, l'extrémité distale de la fibre optique par laquelle sort le rayonnement électromagnétique. On peut également citer tous les traitements endoveineux dans lesquels le rayonnement électromagnétique est produit dans une veine. Pour la lipolyse laser, on pourra se référer par exemple aux publications suivantes: US 6 206 873, US 5 954 710, US 2006/0224148. Pour les traitements endoveineux par laser, on pourra se référer par exemple aux publications US 4 564 011, US 5 531 739, US 6 398 777.

Parmi les traitements de type invasif, on peut également citer tous les traitements thérapeutiques, prophylactiques, ou cosmétiques, consistant à administrer localement dans la partie de corps humain ou animal au moyen d'un instrument de type seringue, une substance ou produit chimique.

Parmi les traitements de type non invasifs, on connaît notamment tous les traitements thérapeutiques, prophylactiques, ou cosmétiques mettant en oeuvre une irradiation externe d'une partie du corps humain ou animal par un rayonnement électromagnétique, par exemple au moyen d'un exo-laser. En particulier, dans le domaine de la dermatologie, sont concernés tous les traitements thermiques de la peau de type non invasif.

Par exemple, il est connu de mettre en oeuvre des traitements thermiques non invasifs pour chauffer le collagène présent dans le derme de la peau.

Une application importante de ces traitements thermiques non invasifs du derme est le remodelage de la peau par le collagène afin de diminuer ou faire disparaître les rides dues au vieillissement, ou de supprimer les aspects inesthétiques de la peau dits « peau d'orange ».

Par exemple dans le brevet américain US 6 659 999 et dans la demande de brevet américain US 2003/0040739, on propose des solutions de remodelage de la peau par le collagène basées sur une irradiation électromagnétique externe de la peau, au moyen d'un exo-laser.

Quel que soit le type de traitement (invasif ou non invasif), l'effet thérapeutique, prophylactique, ou cosmétique dépend des doses de traitement qui sont effectivement délivrées à la partie de corps humain ou animal, mais également de la localisation et la répartition de ces doses. Ces traitements sont ainsi dits dose-dépendants. Un sous dosage peut rendre le traitement thérapeutique, prophylactique, ou cosmétique moins efficace, voire inefficace. A l'inverse, un surdosage peut entrainer des dommages irréversibles dans la zone traitée et par exemple provoquer une destruction irréversible et préjudiciable de certains tissus sains ou cellules saines. Le surdosage ou sous dosage ne dépend pas uniquement de la dose délivrée à chaque position de l'instrument, mais dépend également de la localisation et la répartition de ces doses. La localisation et la répartition des doses dépendent de la manière dont le praticien manipule l'instrument en cours de traitement. En effet, si le praticien qui effectue le traitement commet une erreur de localisation et délivre les bonnes quantités de dose de traitement en différentes positions, mais que tout ou partie de ses positions sont situées en dehors de la zone ou du volume à traiter, ou si il oublie par erreur de traiter une zone ou un volume, ou encore si en cours de traitement il déplace trop rapidement ou au contraire pas assez rapidement l'instrument de délivrance de la dose de traitement de sorte que la répartition des doses délivrées n'est pas correcte, le traitement thérapeutique, prophylactique, ou cosmétique peut être moins efficace ou inefficace, voire dans certains cas peut s'avérer dangereux.

Il est donc primordial pour la réussite et l'innocuité du traitement de pouvoir contrôler de manière efficace non seulement les doses de traitement effectivement délivrées à différentes positions de traitement, mais également de pouvoir contrôler la localisation et la répartition des différentes doses dans un référentiel lié au corps humain ou animal.

Plus particulièrement, une difficulté majeure des traitements par irradiation électromagnétique locale (externe ou interne) d'une partie de corps humain ou animal est liée aux risques de destruction irréversible par effet thermique de cellules non ciblées dans la zone traitée, voire même dans une zone contiguë à la zone traitée. Ce risque dépend non seulement de la puissance et de la longueur d'onde du rayonnement électromagnétique, mais également et surtout de la vitesse à laquelle le spot d'irradiation électromagnétique est déplacé dans la zone à traiter. Or ce dernier paramètre de vitesse de déplacement dépend le plus souvent d'une action manuelle humaine mise en oeuvre par le praticien réalisant le traitement, et est donc une source importante de risques.

Pour tenter de résoudre cette difficulté, on a déjà cherché à ce jour à contrôler l'énergie du rayonnement électromagnétique appliqué lors du traitement. Par exemple, dans la demande de brevet US 2004/0199151, on propose une solution basée sur une mesure de la vitesse de retrait de la fibre optique, et sur un contrôle automatique de la puissance du laser, en fonction de la vitesse mesurée, en sorte de maintenir une énergie de traitement constante appropriée. Différentes solutions de mesure de vitesse de déplacement de la fibre optique sont envisagées. Par exemple, on détecte automatiquement des marques spécifiques faites sur une certaine longueur de la fibre optique, ou on met en oeuvre un dispositif optique de mesure de vitesse dans lequel passe la fibre optique. Cette solution présente deux inconvénients. D'une part, les moyens de mesure de la vitesse de déplacement de la fibre optique sont positionnés dans le champ opératoire, ce qui pose un problème de stérilité de ces moyens de mesure. D'autre part, cette solution ne permet pas une localisation de la zone effectivement traitée dans un référentiel lié au corps humain ou animal, et ne permet pas de connaître la répartition des doses d'énergie dans la zone effectivement traitée.

D'autres solutions de contrôle basées sur une détection extérieure de la température de la peau au moyen par exemple d'un détecteur infrarouge ou de réactifs thermosensibles appliqués sur la peau ont également été proposées. Cependant, ces solutions ne sont pas satisfaisantes, à cause notamment du temps de propagation de la chaleur jusqu'à la surface de la peau. Lorsque le seuil de température de la peau est atteint et détecté, il est généralement trop tard, et des lésions thermiques irréversibles sous-cutanées ont pu déjà être causées.

Dans la demande de brevet internationale WO 2006/107522, il est proposé une solution de lipolyse par laser, dans laquelle le faisceau laser est introduit dans l'hypoderme au moyen d'un ensemble canule/fibre optique. Un objectif dans cette publication est de protéger le derme contre les effets thermiques destructifs du faisceau laser en s'assurant que l'extrémité distale de la fibre optique, lors d'un tir, n'est pas située dans le derme, mais est située dans l'hypoderme en étant suffisamment éloignée du derme. Dans ce but, on contrôle la profondeur du tir laser, en détectant au moyen d'un capteur optique extérieur l'intensité de l'énergie lumineuse du tir, qui traverse les différentes couches (hypoderme, derme, épiderme), et qui est visible par le capteur depuis l'extérieur. Plus l'intensité est élevée, et plus la profondeur du tir laser est faible. Cette solution ne permet pas toutefois une localisation de la zone effectivement traitée dans un référentiel lié au corps humain ou animal, et ne permet pas de connaître la répartition des doses d'énergie dans la zone effectivement traitée.

On a par ailleurs déjà proposé dans la demande de brevet US 2005/0154380, une pièce à main comportant une fibre optique qui est reliée à une source laser, et qui permet un traitement par irradiation électromagnétique d'une partie externe d'un corps humain. Cette pièce à main est en outre équipée de moyens optiques de détection permettant une détection des variations de certains paramètres de positionnement de la pièce à main, tels que des variations de position, des variations d'inclinaison ou des variations de vitesse de déplacement de la pièce à main. Ces informations sont utilisées pour un contrôle automatique de la puissance du laser. Les informations sur les variations des paramètres de positionnement de la pièce à main ne permettent pas de localiser la zone effectivement traitée dans un référentiel lié corps humain ou animal, et de ce fait ne peuvent pas être utilisées pour vérifier que la zone qui devait être traitée a effectivement été traitée, ni pour connaître la répartition des doses d'énergie dans la zone effectivement traitée. En outre, ces moyens de détection sont adaptés uniquement pour une pièce à main non invasive, et ne peuvent pas être utilisés pour une pièce à main invasive.

Il existe par ailleurs également des traitements de type invasif tel que par exemple la liposuccion, au cours desquels on utilise un instrument invasif pour aspirer dans une partie de corps humain ou animal une quantité donnée de cellules ou tissus, tel que par exemple une canule pour aspirer les cellules graisseuses dans le cas particulier de la liposuccion. Ce type de traitement pose les mêmes problèmes que les traitements précités de délivrance d'une dose, et il est important pour l'efficacité et l'innocuité du traitement de pouvoir contrôler non seulement les quantités de cellules ou tissus retirés mais également quelle était la localisation et la répartition, dans un référentiel lié au corps humain ou animal traité, des quantités de cellules ou tissus qui ont été retirées. Par conséquent, dans le présent texte, on assimile à une « dose de traitement », la quantité de cellules ou tissus qui ont été retirés à une position de l'instrument, par analogie avec les traitements précités de délivrance de doses de traitement.

### Objectif de l'invention

Un objectif de l'invention est de proposer une nouvelle solution technique qui permet de faciliter et d'améliorer le contrôle des doses de traitement qui sont délivrées à une partie de corps humain ou animal et/ou qui sont retirées par aspiration dans une partie de corps humain ou animal, et qui peut être mise en oeuvre avec tout type d'instrument utilisé dans un traitement dose-dépendant, c'est-à-dire aussi bien avec un instrument de type invasif ou de type non invasif.

### Résumé de l'invention

L'invention a ainsi pour objet un appareil de traitement d'une partie de corps humain ou animal, comportant un instrument qui permet de délivrer des doses de traitement via une sortie de l'instrument et/ou un instrument qui permet d'aspirer des doses de traitement via une entrée de l'instrument, ledit appareil présentant les caractéristiques techniques visées à la revendication 1.

Selon l'invention, un paramètre de dosimétrie qui est calculé automatiquement est de préférence une cartographie dans un repère prédéfini (pouvant avantageusement être lié au corps humain ou animal traité) des positions absolues des doses délivrées ou aspirées à partir au moins de ladite position absolue instantanée 3D P'[x'(t), y'(t), z'(t)] de la sortie ou entrée de l'instrument. Plus particulièrement encore, mais non nécessairement, cette cartographie est calculée en associant également, à chaque position cartographiée, la dose effective délivrée ou aspirée à ladite position.

Selon l'invention, un autre paramètre de dosimétrie pouvant être calculé est la vitesse de déplacement de la sortie ou de l'entrée de l'instrument utilisé pour délivrer ou aspirer les doses de traitement.

Ledit ou lesdits paramètres de dosimétrie peuvent être affichés sur un écran de contrôle en cours de traitement de manière à permettre au praticien de contrôler le bon déroulement du traitement. Ledit ou lesdits paramètres de dosimétrie peuvent également être enregistrés au cours du traitement, de manière à permettre ultérieurement au praticien de contrôler, une fois les opérations de traitement réalisées, que le traitement s'est déroulé correctement. Ledit ou lesdits paramètres de dosimétrie peuvent également être utilisés pour commander automatiquement le fonctionnement de l'appareil de traitement.

### Brève description des dessins

D'autres caractéristiques de l'invention apparaîtront plus clairement à la lecture de la description détaillée ci-après de plusieurs variantes de réalisation de l'invention données à titre d'exemples non limitatifs et non exhaustifs, laquelle description est faite en référence aux dessins annexés sur lesquels :
- La figure 1 est une représentation schématique d'un exemple d'appareil médical de l'invention permettant un traitement invasif par laser d'une partie de corps humain,
- La figure 2 représente un exemple d'instrument médical comportant une pièce à main permettant la manipulation d'un ensemble canule/fibre optique,
- La figure 3 est un exemple de synoptique des principaux composants électroniques de l'appareil de traitement de la figure 1,
- Les figures 4 et 5 représentent des algorithmes illustrant les principales étapes de fonctionnement de l'appareil de la figure 1.

### Description détaillée

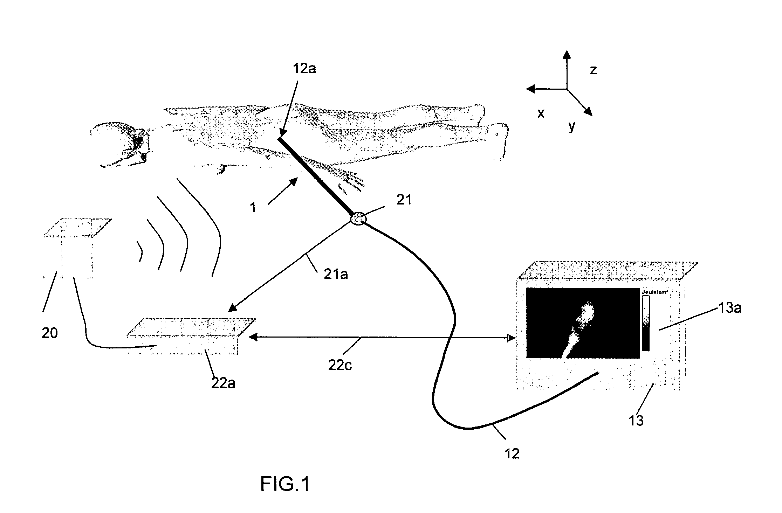

On a représenté de manière schématique sur la figure 1 un exemple d'un appareil médical, qui est conforme à l'invention, et qui permet le traitement d'une partie d'un corps humain C.

Dans cet exemple particulier, mais de manière non limitative et non exhaustive de l'invention, l'appareil de traitement permet la mise en oeuvre de différents types de traitement laser invasif du corps humain. Parmi ces traitements, on peut citer de manière non exhaustive, la lipolyse laser, les traitements lasers endoveineux, le remodelage de la peau par laser, la cicatrisation de la peau par chauffage du collagène présent dans le derme et/ou par stimulation thermique au moyen d'un laser des fibroblastes pour accélérer la production de collagène dans le derme.

### Appareil de traitement

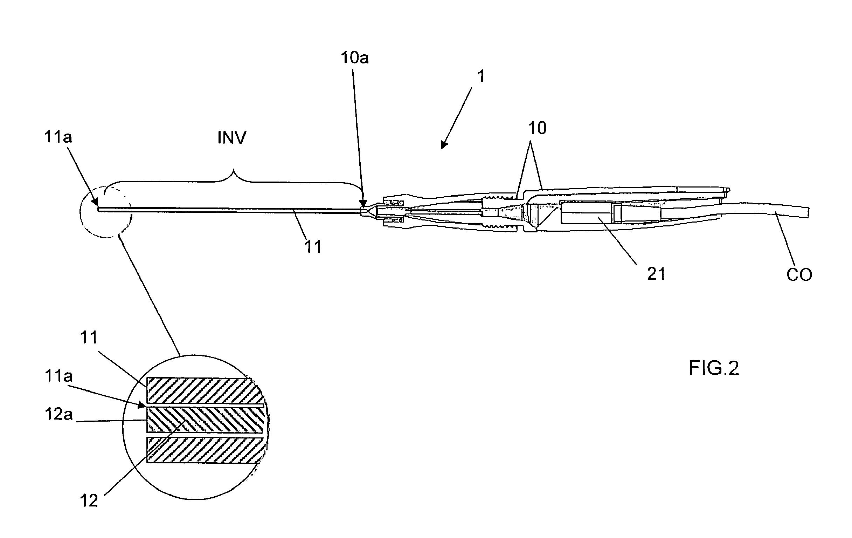

Cet appareil de traitement comporte un instrument 1, qui est manipulable à la main, et qui présente par exemple la structure particulière de la figure 2. En référence à la figure 2, l'instrument 1 comporte par exemple une pièce à main 10 sur laquelle est fixée une canule 11, et une fibre optique 12 qui est enfilée dans la pièce à main 10 et dans la canule 11 et qui est immobilisée par rapport à ladite canule. L'extrémité distale 12a de la fibre optique constitue la sortie de l'instrument 1 par laquelle les doses d'irradiation électromagnétiques sont délivrées. Dans l'exemple particulier illustré sur de la figure 2, l'extrémité distale 12a de la fibre optique 12 affleure avec l'ouverture distale 11a de la canule. Dans une autre variante, l'extrémité distale 12a de la fibre optique 12 pourrait être située à l'extérieur de la canule 11, mais à proximité immédiate de l'ouverture distale 11a de la canule 11.

La pièce à main 10 permet la manipulation à la main de l'ensemble canule 11/fibre optique 12, et constitue une partie non invasive de l'instrument 1. La partie de l'ensemble canule11/fibre optique 12, qui est référencée « INV » sur la figure 2, qui est externe à la pièce à main 10, et qui s'étend depuis l'extrémité distale 10a de la pièce à main 10, constitue une partie invasive de l'instrument 1 destinée à être introduite partiellement ou totalement dans la partie du corps humain C à traiter.

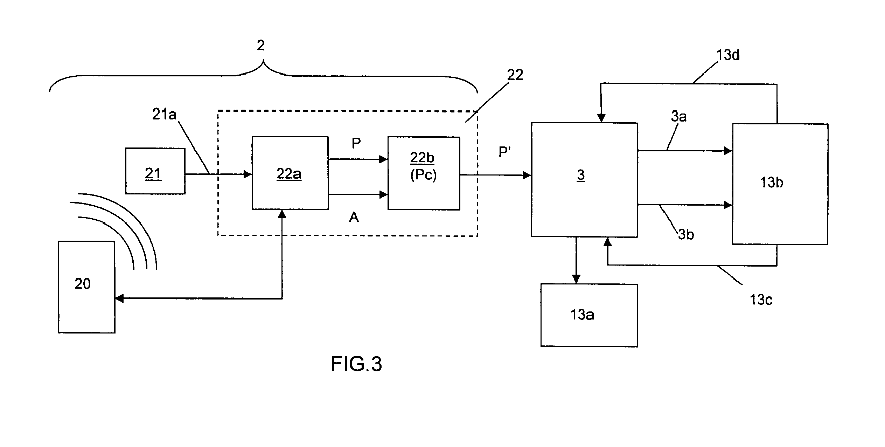

La fibre optique 12 est reliée à son autre extrémité à une source de rayonnement électromagnétique (figure 3 - source laser 13b) qui est intégrée à un dispositif 13 comportant également un écran 13a pour la visualisation du traitement laser. La fréquence d'émission de la source 13b sera choisie de manière connue en soi par l'homme du métier, et peut selon le type de traitement être dans le domaine du visible, de l'infrarouge, des hyperfréquences ou radiofréquences. La fréquence d'émission et/ou la puissance de la source de rayonnement électromagnétique sont de préférence réglables. Lorsque la source de rayonnement électromagnétique fonctionne, l'instrument 1 délivre à son extrémité distale d'émission 12a un rayonnement électromagnétique pouvant être appliqué à une partie du corps C à traiter.

En référence aux figures 1 et 3, l'appareil de traitement comporte également :
- des moyens 2 de localisation de la position absolue instantanée 3D P'(x'(t), y'(t), z'(t)) de la sortie 12a de la fibre optique 12 dans un repère tridimensionnel prédéfini (X,Y,Z),
- et des moyens électroniques 3 de contrôle de dosimétrie qui sont aptes à calculer au moins un paramètre de dosimétrie à partir au moins de ladite position absolue instantanée P'(x'(t), y'(t), z'(t)).

Un exemple particulier de réalisation des moyens de localisation 2 et des moyens électroniques de contrôle de dosimétrie 3 va à présent être détaillé.

### Moyens de localisation 2

En référence aux figures 1 et 3, les moyens de localisation 2 comportent un émetteur de champ magnétique 20, un capteur 21 qui est fixé à l'instrument 1, à une position différente de la position de la sortie 12a de la fibre optique 12, et des moyens électroniques de calcul 22.

Plus particulièrement, en référence à l'exemple particulier de la figure 2, le capteur 21 est logé à l'intérieur de la pièce à main 10. Sur cette figure 2, on a représenté un cordon électrique CO qui est connecté au capteur 21, et qui contient d'une part les fils électriques d'alimentation du capteur et d'autre part les fils électriques transportant les signaux électriques 21 a délivrés par le capteur 21.

En fonctionnement, l'émetteur 20 est fixe et positionné à proximité du corps C et émet un champ magnétique qui est reçu par le capteur 21. Le capteur 21 est sensible au champ magnétique produit par l'émetteur 20 et délivre des signaux électriques 21a qui sont caractéristiques de sa position absolue instantanée et de son inclinaison absolue instantanée dans ledit champ magnétique. Ces signaux électriques 21 a sont reçus et traités par des premiers moyens électroniques de calcul 22a qui sont aptes calculer en temps réel des données P[x(t), y(t), z(t)]) codant la position absolue instantanée 3D dudit capteur et des données A[α(t), β(t), θ(t)] codant l'inclinaison absolue instantanée 3D dudit capteur dans un repère tridimensionnel prédéfini (X, Y, Z).

Le repère tridimensionnel prédéfini (X,Y, Z) est le référentiel de l'émetteur de champ magnétique 20. Lors d'un traitement, le corps C est placé dans le champ magnétique émis par cet émetteur 20, dans une position connue et la partie de corps C traitée est immobile dans le repère (X,Y,Z) pendant la durée de traitement. Le repère tridimensionnel prédéfini (X,Y,Z) est ainsi lié à la partie de corps C à traiter.

En référence à la figure 3, les données P[x(t), y(t), z(t)] sur la position absolue instantanée 3D du capteur 21 et les données A[α(t), β(t), θ(t)] sur l'inclinaison absolue instantanée 3D du capteur 21 sont traitées en temps réel par des deuxièmes moyens électroniques de calcul 22b, qui sont paramétrés avec la position relative Pc(dx,dy,dz) du capteur 21 par rapport à la sortie 12a de la fibre optique 12. Cette position relative Pc du capteur 21 est fixe dans le temps, quelles que soient la position et l'inclinaison de l'instrument 1, et est une information, de préférence modifiable, qui est par exemple stockée dans une mémoire des deuxièmes moyens électroniques de calcul 22b.

Les deuxièmes moyens électroniques de calcul 22b sont conçus de manière à calculer en temps réel des données P'[x'(t), y'(t), z'(t)] codant la position absolue instantanée 3D de la sortie 12a de la fibre optique 12 de l'instrument 1 dans le repère tridimensionnel (X,Y,Z), à partir desdites données codant la position absolue instantanée 3D P[X(t), Y(t), Z(t)] et l'inclinaison absolue instantanée 3D A[α(t), β(t), θ(t)] du capteur 21, et à partir de la position relative Pc(dx,dy,dz) du capteur 21.

Dans la variante particulière de réalisation des figures 1 et 3, les premiers moyens électroniques de calcul 22a sont intégrés dans un boîtier externe distinct du dispositif précité 13 et les deuxièmes moyens électroniques de calcul 22b sont intégrés audit dispositif 13 et communiquent localement avec les premiers moyens électroniques de calcul 22a par une liaison 22c (figure 1) qui peut indifféremment être de type filaire ou sans fil.

L'émetteur 20, le capteur 21 et les premiers moyens électroniques de calcul 22a sont des moyens connus, et peuvent par exemple, et de manière non limitative de l'invention, être constitués par les composants d'un appareil de localisation magnétique commercialisé par la société Ascension Technology Corporation sous la marque « Flock of Birds® ». Les deuxièmes moyens électroniques de calcul 22b peuvent être constitués par toute unité de traitement programmable, mettant en oeuvre par exemple microprocesseur ou microcontrôleur apte à exécuter un programme de calcul permettant de réaliser le calcul d'une position absolue instantanée 3D P'[x'(t), y'(t), z'(t)] à partir des données de position absolue instantanée 3D P[x(t), y(t), z(t)] et d'inclinaison absolue instantanée 3D A[α(t), β(t), θ(t)].

Dans une autre variante de réalisation, les deuxièmes moyens électroniques de calcul 22b pourraient être intégrés dans le même boitier que les premiers moyens électroniques de calcul 22a. Dans une autre variante de réalisation, les premiers 22a et deuxièmes 22b moyens électroniques de calcul pourraient être réalisés en utilisant le même processeur de calcul.

### Moyens électroniques de contrôle de dosimétrie 3

Les moyens électroniques de contrôle de dosimétrie 3 peuvent être implémentés sous la forme de tout type d'unité électronique de traitement programmable comportant notamment un microprocesseur ou microcontrôleur apte à exécuter automatiquement un programme stocké dans une mémoire et spécifique de l'invention.

Les moyens électroniques de contrôle de dosimétrie 3 reçoivent en entrée au moins des données P'[x'(t), y'(t), z'(t)] codant la position absolue instantanée 3D de la sortie 12a de la fibre optique 12, et également dans l'exemple particulier illustré deux signaux 13c et 13d délivrés par la source laser 13b. Le signal 13c permet aux moyens électroniques de contrôle de dosimétrie 3 d'être informés si un tir laser est en cours ou non. Ce signal 13c est par exemple un signal électrique de type binaire qui peut prendre deux niveaux haut et bas, et qui est par exemple à l'état haut lorsqu'un un tir est en cours (source laser activée) et à l'état bas 0 dans le cas contraire. Le signal 13c est un signal codant la puissance instantanée PUI(t) de la source laser 13b.

Les moyens électroniques de contrôle de dosimétrie 3 sont programmés pour calculer des paramètres de dosimétrie, dont par exemple la cartographie des doses délivrées et la vitesse de déplacement de la sortie 12a de la fibre optique 12 de l'instrument 1, et pour afficher ces paramètres de dosimétrie sur l'écran 13a afin de permettre un contrôle visuel du traitement par le praticien.

Dans l'exemple particulier de réalisation de la figure 3, et de manière optionnelle selon l'invention, les moyens électroniques de contrôle de dosimétrie 3 délivrent également en sortie deux signaux de contrôle 3a, 3b leur permettant de commander la source laser 13b. Le signal 3a est un signal de réglage de la puissance de la source laser 13b. Le signal 3b est un signal permettant de commander l'arrêt de la source laser 13b, en cas de détection d'un surdosage.

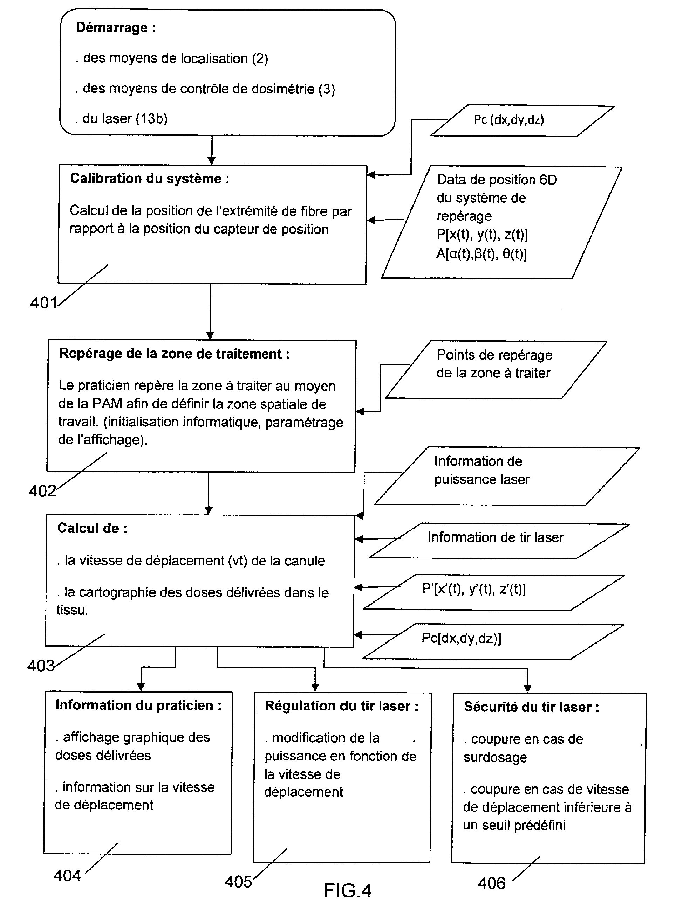

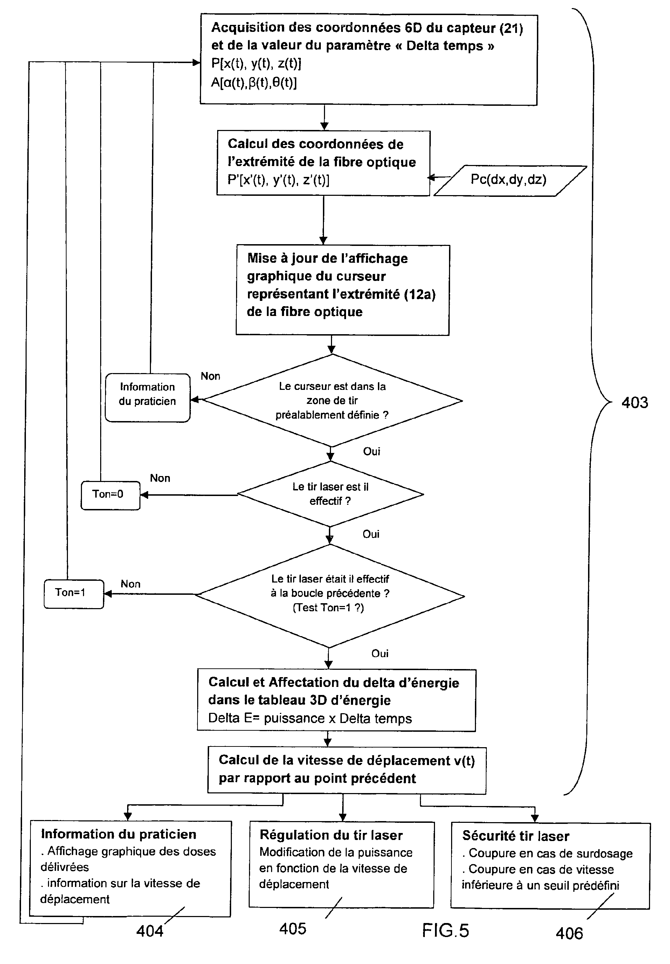

Les fonctionnalités des moyens 2 de localisation et des moyens électroniques de contrôle de dosimétrie 3 vont à présent être détaillées à la lumière des algorithmes de fonctionnement des figures 4 et 5, donnés à titre d'exemples non limitatifs et non exhaustifs de l'invention.

### Algorithmes des figures 4 et 5

Après démarrage des moyens de localisation 2, des moyens électroniques de contrôle de dosimétrie 3 et de la source laser 13b, les moyens de localisation 2 mettent en oeuvre une première étape de calibration 401 (figure 4). Cette étape de calibration consiste à paramétrer la position relative Pc(dx,dy,dz) du capteur 21 par rapport à la sortie 12a de la fibre optique 12, de sorte que les deuxièmes moyens de calcul 22b puissent calculer automatiquement la position instantanée 3D P'[x'(t), y'(t), z'(t)] de la sortie 12a de la fibre optique 12, à partir des données fournis par les premiers moyens électroniques de calcul 22a et codant la position absolue instantanée 3D P[X(t), Y(t), Z(t)] et l'inclinaison absolue instantanée 3D A[α(t), β(t), θ(t)] du capteur 21

Une fois les moyens de localisation 2 calibrés, les moyens de contrôle de dosimétrie 3 exécutent un programme de repérage de la zone de traitement (figure 4- Etape 402).

Ce programme consiste à faire entrer par le praticien plusieurs points de repérage, en utilisant l'instrument 1, afin de délimiter la zone de traitement. Ce repérage est par exemple réalisé de manière non invasive, en appliquant la sortie 12a de l'instrument au contact externe du corps C, de manière à délimiter une zone de traitement en deux dimensions. Ces points de repérage sont utilisés pour l'affichage sur l'écran 13a de la zone à traiter.

Une fois ces étapes de calibration 401 et de repérage 402 terminées, l'appareil de traitement est prêt à être utilisé par le praticien.

Le praticien réalise au moyen de l'instrument 1 le traitement laser sous-cutané ou intra-cutané approprié de manière connue en soi, selon un protocole de traitement qu'il a préalablement déterminé. Au cours de ce traitement, le praticien pratique un incision dans la peau et introduit l'extrémité de l'ensemble canule 11/fibre optique 12 sous le plan dermique derme ou dans le derme (selon le type de traitement) dans la zone de traitement qui a été repérée. Puis le praticien réalise de manière connue en soi le traitement en effectuant une série de tirs laser et en déplaçant l'ensemble canule 11/fibre optique 12 dans la zone de traitement.

Les moyens de contrôle de dosimétrie 3 sont programmés pour en cours de traitement, calculer automatiquement deux paramètres de contrôle de dosimétrie (étape 403) : la vitesse de déplacement de la sortie 12a de la fibre optique 12 , ce qui est équivalent dans cet exemple particulier d'instrument 1 à la vitesse de déplacement de la canule 11 ; la cartographie des doses d'énergie laser délivrées dans le tissu, c'est-à-dire les doses d'énergie laser délivrées en chaque point de la zone de traitement.

La figure 5 montre un algorithme plus détaillé pour la mise en oeuvre de l'étape 403.

Le calcul de la cartographie des doses d'énergie laser est effectué pour chaque tir laser et de manière itérative tant que le tir laser est effectif. Cette cartographie consiste à associer, dans un tableau 3D, à chaque position absolue instantanée 3D P'[X'(t), Y'(t), Z'(t)] de la sortie 12a de la fibre optique, la dose de rayonnement électromagnétique délivrée à cette position, c'est à dire la somme des énergies délivrée à cette position (sommation des valeurs calculée du paramètre « Delta E » de la figure 5 pour chaque position P'[X'(t), Y'(t), Z'(t)]). Ainsi cette cartographie des doses d'énergie laser est calculée à partir des informations suivantes :
- les données P'[x'(t), y'(t), z'(t)] codant la position instantanée 3D de la sortie 12a de la fibre optique 12 ;
- l'information qu'un tir laser est en cours, cette information étant fournie aux moyens de contrôle de dosimétrie 3 par le signal 13c ;
- la puissance du laser (paramètre « Puissance » sur la figure 5), cette information étant fournie aux moyens de contrôle de dosimétrie 3 par le signal 13d ;
- la durée d'émission du rayonnement (paramètre « delta temps » sur la figure 5)

La cartographie des doses d'énergie laser délivrées dans le tissu peu également être une cartographie 2D (sommation des doses d'énergie (paramètre « Delta E » sur la figure 5) dans un plan prédéfini (par exemple le plan X,Y) ou une cartographie 1 D (sommation des doses d'énergie (paramètre « Delta E » sur la figure 5) suivant un axe prédéfini (par exemple l'axe X).

Dans une variante de réalisation plus simple, la cartographie des doses pourrait être établie en enregistrant uniquement les positions absolues successives des doses délivrées ( paramètre P'[x'(t), y'(t), z'(t)]) sans calcul d'énergie ( paramètre « Delta E »)

Le calcul de la vitesse de déplacement est effectué à partir des positions instantanées 3D P'[x'(t), y'(t), z'(t)] de la sortie 12a de la fibre optique 12 qui sont successivement calculées à chaque itération.

Ces paramètres de dosimétrie (cartographie des doses et vitesse de déplacement de la sortie 12a de la fibre optique 12) sont en outre utilisés par les moyens de contrôle de dosimétrie 3 de la manière suivante.

La vitesse de déplacement et la cartographie des doses d'énergie laser sont affichées en temps réel sur un écran 13a du dispositif 13 (figure 4 ou 5/ étape 404), ce qui permet au praticien de contrôler visuellement le bon déroulement du traitement laser. La vitesse peut être affichée sous forme numérique ou être codée par exemple au moyen d'une échelle de type barre graphe. Pour la cartographie, la dose en chaque point sera par exemple codée par une couleur en fonction du niveau de dose.

Dans une variante de réalisation, les moyens de contrôle de dosimétrie 3 peuvent avantageusement être conçus (figure 4 ou 5/ étape 405), pour commander automatiquement la puissance de la source laser 13b, au moyen du signal de réglage précité 3a, en fonction de la vitesse de déplacement de la canule 11 qui est calculée.

Dans une variante de réalisation, les moyens de contrôle de dosimétrie 3 peuvent avantageusement être conçus (figure 4 ou 5/ étape 406)
- pour comparer automatiquement la dose délivrée par unité de surface ou par unité de volume avec au moins un seuil prédéfini, et
- pour commander automatiquement la coupure de la source laser 13b, au moyen du signal de commande précité 3b, en cas de détection d'un dépassement de ce seuil (détection automatique d'un surdosage).
   Dans une variante de réalisation, les moyens de contrôle de dosimétrie 3 peuvent avantageusement être conçus (figure 4 ou 5/ étape 406)
- pour comparer automatiquement la vitesse de déplacement de la canule 11 qui est calculée à l'étape 403 avec au moins un seuil prédéfini, et
- pour commander automatiquement la coupure de la source laser 13b, au moyen du signal de commande précité 3b, lorsque la vitesse de déplacement de la canule 11 qui est calculée à l'étape 403 est inférieure à ce seuil (détection automatique d'un surdosage).

Les deux modes précités de détection automatique d'un surdosage peuvent avantageusement être mis en oeuvre dans une même variante de réalisation.

Grâce à ces moyens de contrôle de dosimétrie 3, le traitement peut être réalisé par le praticien avec une plus grande sécurité. En outre la cartographie de doses délivrées peut être enregistrée, et éventuellement utilisée par le praticien pour un suivi dans le temps des protocoles de traitement d'un patient.

L'invention n'est pas limitée à un appareil pour la mise en oeuvre d'un traitement par irradiation électromagnétique (visible, infrarouge, hyperfréquences ou radiofréquences) intra-cutanée ou sous-cutanée, mais peut également être mise en oeuvre pour contrôler les doses d'énergie délivrées au moyen d'un appareil comportant un instrument adapté pour la mise en oeuvre d'un traitement endoveineux, ou comportant un instrument de type exo-laser adapté pour la mise en oeuvre d'un traitement laser externe non invasif appliqué sur la surface de la peau.

La source d'énergie 13b n'est pas nécessairement une source de rayonnement électromagnétique, mais peut par exemple être une source d'énergie acoustique, l'instrument étant dans ce cas conçu pour délivrer l'énergie acoustique produite par ladite source.

L'invention n'est pas non plus limitée à un appareil de traitement par laser, mais peut plus généralement être mise en oeuvre pour contrôler tout type de doses de traitement délivrées au moyen de tout type connu d'instrument médical, les doses pouvant par exemple être un produit chimique ou médicament administré à une partie de corps humain ou animal.

L'invention peut également être mise en oeuvre pour des traitements de type invasif, tel que par exemple la liposuccion, au cours desquels on utilise un instrument invasif pour aspirer dans une partie de corps humain ou animal une quantité donnée de cellules ou tissus, tel que par exemple une canule pour aspirer les cellules graisseuses dans le cas particulier de la liposuccion. Dans ce cas, les moyens électronique de localisation permettent de localiser automatiquement la position absolue instantanée 3D (X'(t), Y'(t), Z'(t)) de l'entrée de l'instrument dans un repère tridimensionnel prédéfini, et le calcul des doses par les moyens électroniques de contrôle dosimétrie 3 correspond à la quantité de cellules ou tissus qui ont été retirés à une position de l'instrument.

L'invention peut également être appliquée à un appareil de traitement qui à la fois permet de délivrer des doses de traitement via une sortie 12a d'un instrument et permet d'aspirer des doses de traitement via une entrée d'un instrument (même instrument ou instrument différent de celui délivrant les doses). Dans ce cas, les moyens de localisation permettent de préférence de localiser dans le repère tridimensionnel (X,Y,Z) la position absolue instantanée 3D de la sortie de l'instrument délivrant les doses et la position absolue instantanée 3D de l'entrée de l'instrument aspirant les doses, et sont ainsi aptes à délivrer des données P'[(x'(t), y'(t), z'(t)] codant la position absolue instantanée 3D de la sortie de l'instrument délivrant les doses, et des données P'[(x'(t), y'(t), z'(t)] codant la position absolue instantanée 3D de l'entrée de l'instrument aspirant les doses.

## Revendications

1. Appareil de traitement d'une partie de corps humain ou animal, comportant un instrument (1) qui permet de délivrer des doses de traitement via une sortie (12a) de l'instrument et/ou comportant un instrument (1) qui permet d'aspirer des doses de traitement via une entrée de l'instrument, **caractérisé en ce qu'**il comprend des moyens (2) de localisation, dans un repère tridimensionnel prédéfini (X,Y,Z), de la position absolue instantanée 3D de la sortie (12a) de l'instrument délivrant les doses et/ou de l'entrée de l'instrument (12) aspirant les doses, lesquels moyens (2) de localisation sont aptes à délivrer des données P'[(x'(t), y'(t), z'(t)] codant la position absolue instantanée 3D de la sortie (12a) et/ou de l'entrée de l'instrument (12), et **en ce qu'**il comprend des moyens électroniques (3) de contrôle de dosimétrie qui sont aptes à calculer au moins un paramètre de dosimétrie à partir au moins des données de localisation P'[(x'(t), y'(t), z'(t)] qui sont délivrées par les moyens (2) de localisation.

2. Appareil selon la revendication 1, **caractérisé en ce que** les moyens électroniques (3) de contrôle de dosimétrie sont aptes à calculer au moins un paramètre de dosimétrie à partir au moins des données de localisation P'[(x'(t), y'(t), z'(t)] qui sont délivrées par les moyens (2) de localisation et à partir d'au moins un paramètre de fonctionnement des moyens (13b) de délivrance des doses de traitement et/ou d'aspiration des doses de traitement.

3. Appareil selon la revendication 1 ou 2, **caractérisé en ce que** le paramètre de fonctionnement des moyens (13b) de délivrance des doses de traitement et/ou d'aspiration des doses de traitement, qui est utilisé par les moyens électroniques (3) de contrôle de dosimétrie pour le calcul d'au moins un paramètre de dosimétrie, est la puissance desdits moyens (13b).

4. Appareil selon l'une des revendications 1 à 3, **caractérisé en ce que** les moyens électroniques (3) de contrôle de dosimétrie sont aptes à calculer dans un repère prédéfini (X,Y,Z) un paramètre de dosimétrie, qui est une cartographie des positions absolues des doses délivrées ou aspirées, à partir au moins des données de localisation P'[(x'(t), y'(t), z'(t)] qui sont délivrées par les moyens (2) de localisation.

5. Appareil selon la revendication 4, **caractérisé en ce que** les moyens électroniques (3) de contrôle de dosimétrie sont aptes à calculer ladite cartographie en associant également, à chaque position cartographiée, la dose effective délivrée ou aspirée à ladite position.

6. Appareil selon l'une des revendications 1 à 5, **caractérisé en ce que** les moyens électroniques de contrôle de dosimétrie (3) sont aptes à calculer un paramètre de dosimétrie qui est la vitesse de déplacement v(t) de la sortie ou de l'entrée de l'instrument, à partir des données de localisation P'[(x'(t), y'(t), z'(t)] qui sont délivrées par les moyens (2) de localisation.

7. Appareil selon l'une des revendications 1 à 6, **caractérisé en ce que** lesdits moyens de localisation (2) comportent des moyens de détection (20, 21) et des moyens électroniques de calcul (22), **en ce que** les moyens de détection (20, 21) comportent au moins un capteur (21), qui est fixé à l'instrument (1) à une position différente de la position de la sortie (12a) ou de l'entrée de l'instrument, et sont aptes à fournir, aux moyens électroniques de calcul (22), des données P[x(t), y(t), z(t)] codant la position absolue instantanée 3D dudit capteur et des données A[α(t), β(t), θ(t)] codant l'inclinaison absolue instantanée 3D dudit capteur dans un repère tridimensionnel prédéfini (X, Y, Z), et **en ce que** les moyens électroniques de calcul (22) sont aptes à calculer la position absolue instantanée 3D P'[x'(t), y'(t), z'(t)] de la sortie (12a) ou de l'entrée de l'instrument à partir desdites données de position absolue instantanée 3D P[x(t), y(t), z(t)] et d'inclinaison absolue instantanée 3D A[α(t), β(t), θ(t)] du capteur (21), et à partir de la position relative Pc (dx,dy,dz) du capteur par rapport à la sortie (12a) ou à l'entrée de l'instrument.

8. Appareil de traitement selon la revendication 7, **caractérisé en ce que** les moyens de détection comportent des moyens d'émission (21) d'un champ magnétique, et **en ce que** le capteur (21) des moyens de détection est sensible au champ magnétique produit par lesdits moyens d'émission (20) et est apte à délivrer des signaux électriques (21 a) qui sont caractéristiques de sa position absolue instantanée et de son inclinaison absolue instantanée dans ledit champ magnétique.

9. Appareil selon l'une des revendications 7 ou 8, **caractérisé en ce que** l'instrument (1) comporte une partie (INV) dite invasive, qui comprend la sortie (12a) ou l'entrée de l'instrument et qui est adaptée pour être introduite dans une partie de corps humain ou animal, et **en ce que** ledit capteur (21) des moyens de détection est positionné dans une autre partie non invasive de l'instrument.

10. Appareil selon l'une des revendications 1 à 9, **caractérisé en ce que** l'instrument (1) comporte une fibre optique (12) qui est reliée à une source de rayonnement électromagnétique (13b), et qui permet de délivrer à son extrémité distale d'émission (12a) le rayonnement électromagnétique produit par ladite source, ladite extrémité distale (12a) de la fibre optique correspondant à la sortie de l'instrument.

11. Appareil de traitement selon la revendication 10 et l'une des revendications 7 à 9, **caractérisé en ce que** l'instrument (1) comprend une canule (11) qui est enfilée et immobilisée sur la fibre optique (12), et **en ce que** le capteur (21) des moyens de détection est solidaire de l'ensemble canule (11)/fibre optique (12).

12. Appareil selon l'une des revendications 1 à 9, **caractérisé en ce qu'**il comprend une source d'énergie acoustique, et **en ce que** l'instrument est apte à délivrer l'énergie acoustique produite par ladite source.

13. Appareil selon l'une des revendications 1 à 12, **caractérisé en ce qu'**il comprend des moyens d'affichage (13a) du ou des paramètres de dosimétrie qui sont calculés par les moyens électroniques (3) de contrôle de dosimétrie.

14. Appareil selon l'une des revendications 1 à 13, **caractérisé en ce que** les moyens électroniques (3) de contrôle de dosimétrie sont en outre aptes à commander les moyens (13b) de délivrance des doses de traitement et/ou d'aspiration des doses de traitement, de manière à régler automatiquement la dose de traitement délivrée et/ou la dose de traitement aspirée en fonction du ou des paramètres de dosimétrie calculés.

15. Appareil selon l'une des revendications 1 à 14, **caractérisé en ce que** les moyens électroniques (3) de contrôle de dosimétrie sont aptes à détecter automatiquement un surdosage à partir du ou des paramètres de dosimétrie calculés, et sont aptes à commander les moyens (13b) de délivrance des doses de traitement et/ou d'aspiration des doses de traitement, de manière à arrêter automatiquement les moyens (13b) de délivrance des doses de traitement et/ou d'aspiration des doses de traitement en cas de détection d'un surdosage.
